# EUROPÄISCHE PATENTSCHRIFT

(11) **EP 0 846 006 B1**
(45) Veröffentlichungstag und Bekanntmachung des Hinweises auf die Patenterteilung: **06.05.1999**
(21) Anmeldenummer: 96942241.9
(22) Anmeldetag: 23.08.1996
(51) Int. Cl.: A61M 1/02

(54) **VERFAHREN UND VORRICHTUNG ZUM FLIESSTRENNEN VON VOLLBLUT ALS GEMISCH VON FLÜSSIGKEITEN IN EINZELNE VERSCHIEDENFARBIGE BLUTBESTANDTEILE, INSBESONDERE ZUR SEPARATION VON THROMBOZYTENKONZENTRAT AUS BUFFYCOAT**
PROCESS AND DEVICE FOR FLUID SEPARATION OF WHOLE BLOOD AS A MIXTURE OF LIQUIDS INTO INDIVIDUAL, DIFFERENTLY-COLORED BLOOD CONSTITUENTS, IN PARTICULAR FOR SEPARATION OF CONCENTRATED THROMBOCYTES FROM BUFFY COAT
PROCEDE ET DISPOSITIF POUR SEPARER PAR FLUAGE DU SANG COMPLET SOUS FORME DE MELANGE DE LIQUIDES, EN CONSTITUANTS SANGUINS INDIVIDUELS DE DIFFERENTES COULEURS, NOTAMMENT POUR SEPARER UN CONCENTRE DE THROMBOCYTES D'UNE COUCHE LEUCO-PLAQUETTAIRE

(30) Priorität: 23.08.1995 DE 19530969
(43) Veröffentlichungstag der Anmeldung: 10.06.1998
(73) Patentinhaber: DEUTSCHES ROTES KREUZ BLUTSPENDEDIENST BADEN-WÜRTTEMBERG GEMEINNÜTZIGE GMBH, 76530 Baden-Baden (DE)
(72) Erfinder: SPINDLER, Jörg, D-69126 Heidelberg (DE)
(74) Vertreter: Mierswa, Klaus, Dipl.-Ing.
(86) Internationale Anmeldenummer: DE9601570
(87) Internationale Veröffentlichungsnummer: WO9707836

(56) Entgegenhaltungen:
- EP-A- 0 329 786
- EP-A- 0 611 579
- EP-A- 0 682 953
- WO-A-89/01796
- FR-A- 2 620 950
- US-A- 3 900 396
- US-A- 4 085 047
- US-A- 4 227 814

## Beschreibung

### Technisches Gebiet:

Die Erfindung betrifft Verfahren zum Fließtrennen von Vollblut als Gemisch von Flüssigkeiten in einzelne verschiedenfarbige Blutbestandteile, welches in flexible Behältnisse, insbesondere Beutel, abgepackt ist, die untereinander durch eine wenigstens zum Teil lichttransparente Verbindung, insbesondere flexibler Schlauch, miteinander verbunden sind, und die Blutbestandteile bei Krafteinwirkung von einem Behältnis durch die lichttransparente Verbindung in ein anderes Behältnis fließen, insbesondere zur Separation von Thrombozytenkonzentrat aus Buffycoat, gemäß dem Oberbegriff von Anspruch 1 und 3, sowie Vorrichtungen zur Durchführung des Verfahrens.

### Stand der Technik:

Es gibt verschiedene Techniken zum Trennen von Vollblut in seine unterschiedlichen Bestandteile, insbesondere zur Gewinnung eines Thrombozytenkonzentrats. Allen Techniken ist wenigstens eine Zentrifugation, zur Gewinnung des Thrombozytenkonzentrats und eine wenigstens teilweise manuelle Handhabung gemeinsam. Das Ausgangsprodukt ist Vollblut, welches sich in einem flexiblen Beutel befindet, der über transparente flexible Schläuche mit weiteren Satellitenbeuteln luft- und flüssigkeitsdicht verbunden ist, die zur Aufnahme der getrennten Konzentrate dienen.

Nachdem aus dem mit Vollblut gefüllten Beutel mittels einer Presse der Hauptanteil an Plasma und Erythrozyten entfernt worden ist, wird zur Gewinnung eines Thrombozytenkonzentrats das verbleibende Gemisch aus Erythrozyten- und Leukozytenzellen, Thrombozytenplättchen sowie eines Restes an Plasma nochmals zentrifugiert und dieses sogenannte Buffycoat wiederum in eine Presse gestellt, um eine Trennung der Erythrozyten und Leukozyten vom nunmehr thrombozytenreichen Plasma durchzuführen. Beim Abpreßvorgang steuert die Bedienungsperson mittels einer Klemme an einem durchsichtigen Verbindungsschlauch den Durchfluß des abgedrückten thrombozytenreichen Plasmas und beobachtet dabei, wann nach dem durchsichtig-gelblichen Plasma die ersten Erythrozyten und Leukozyten im Verbindungsschlauch auftauchen. Falls dies geschieht, klemmt die Bedienungsperson den Schlauch ab, entlüftet anschließend den Thrombozytenbeutel und verschweißt das Schlauchende.

Dabei ist darauf zu achten, daß keine Erythrozyten- und Leukozytenzellen in den Thrombozytenbeutel gelangen, aber auch, daß sämtliches Thrombozytenplättchen-Plasma-Gemisch aus dem ursprünglichen Beutel gewonnen wird.

Durch die Klemme, die den Verbindungsschlauch umfaßt, kann die Bedienungsperson manuell die Fließgeschwindigkeit innerhalb des Schlauches steuern. Ist die Fließgeschwindigkeit zu hoch, kommt es zum sogenannten Siphoneffekt, d.h. die erheblich größeren Erythrozyten- und Leukozytenzellen werden aus der Buffycoatschicht herausgerissen und verunreinigen das Thrombozytenkonzentrat. Es ist für die Bedienungsperson schwierig, eine gleichmäßige Durchflußgeschwindigkeit zu erzielen. Bei gleichmäßigem Durchlauf und der normalen Beutelgröße würde dies ungefähr 140 Sekunden dauern, d.h. die Bedienungsperson müßte die Klemme ca. 140 sec gleichmäßig gedrückt halten, was eine hohe Konzentration erfordert. Um diese Zeit abzukürzen, läßt die Bedienungsperson das Plasma-Thrombozytenkonzentrat am Anfang meistens schneller laufen und zum Schluß langsamer, was allerdings eine größere Verunreinigung durch Leukozythen- und Erythrozytenzellen durch den Siphoneffekt bedeutet und zu einer nicht optimalen Ausbeute des Thrombozytenplasmas führt, weil die Buffycoatschicht aufgewirbelt wurde. Am Ende des Abpreßvorganges muß die Bedienungsperson visuell beobachten, wann im Schlauch die ersten Erythrozyten ankommen. Die Bedienungsperson kann aber die ersten Erythrozytenzellen gar nicht sehen, da sie noch in einer zu geringen Konzentration vorkommen, geschweige denn, kann die Bedienungsperson die praktisch farblosen Leukozythenzellen optisch wahrnehmen. Das heißt, daß je nach Tagesform und Aufmerksamkeit der Bedienungsperson der Schlauch zu früh oder zu spät abgeklemmt wird. Bei zu früher Abklemmung bedeutet das eine zu geringe Thrombozytenausbeute bis hin zur Aussortierung, da die Thrombozytenkonzentration > 0,5x10¹¹ betragen muß. Oder das Gewicht von 56 Gramm netto der Plasma-Thrombozytenausbeute wird unterschritten. Wenn der Schlauch zu spät abgeklemmt wird, befinden sich zu viele Leukozythen und Erythrozyten im Präparat (Grenze < 1,0 x 10⁸ WBC < 2,0 x 10⁹ RBC).

Die manuelle Thrombozytenplättchengewinnung ist deshalb mit einer mehr oder weniger großen Verunreinigung von Erythrozyten und Leukozyten verbunden, weshalb die Gleichmäßigkeit der Qualität der Thrombozytenkonzentrate gewissen Schwankungen unterworfen ist. Die Bedienungsperson entscheidet, bis zu welcher Verfärbung, d.h. Verunreinigung, des Serums sie die Thrombozytenkonzentrate zuläßt. Dabei dürfen lipämische und stark verfärbte, insbesondere rote Plasmen nicht verwendet werden, weil z.B. in den roten Plasmen Erythrozytenfragmente und Hämoglobin vorhanden sein können, die sich nicht abzentrifugieren lassen. Das bedeutet, daß nicht völlig auszuschließen ist, daß die Bedienungsperson unzulässige Thrombozytenkonzentrate zulassen kann.

Durch die DE 38 15 643 C1 ist eine Vorrichtung zur Trennung von Gesamtblut in einem flexiblen, durchsichtigen Beutel mit mindestens einer Auslaßleitung und Absperreinrichtung bekannt, bestehend aus einem Gehäuse mit einer Frontplatte und einer relativ zur Frontplatte schwenkbaren Andruckplatte mit einer Antriebseinrichtung für die Andruckplatte sowie einer Abtasteinrichtung. Die Abtasteinrichtung weist einen optischen Detektor auf, der ein Schlauchdetektor im Schlauchbereich sein kann und der eine Absperreinrichtung für eine der Auslaßeinrichtungen betätigt, wenn eine bestimmte Komponentenschicht ein vorgegebenens Niveau im Beutel erreicht. Weiterhin sind aus der GB-PS 15 37 096 Schlauchdetektoren als Abtasteinrichtungen bekannt.

Durch die FR 2620950 ist eine Vorrichtung zum Trennen von Blut bekannt, welches in flexible Behältnissen, insbesondere Beuteln, abgepackt ist, die untereinander durch einen lichttransparenten, flexiblen Schlauch verbunden sind, wobei die Blutbestandteile bei Krafteinwirkung von einem Behältnis durch die lichttransparente Verbindung in ein anderes Behältnis fließen. Das Ausgangssignal einer optisch-elektrischen Fotometereinheit, an der die Flüssigkeit innerhalb der lichttransparenten Verbindung vorbeifließt, dient zur Steuerung einer Unterbrechereinheit, welche den Blutfluß abklemmt. Mit dieser Vorrichtung gelingt zwar, eine weitgehend bedienerunabhängige Trennung von Blut anhand des optischen Kontrasts der einzelnen Blutkomponenten durchzuführen, jedoch ist die Reinheit bzw. optimale Ausbeute der so separierten Komponenten nicht unbedingt gewährleistet, da die unterschiedlichen Eigenfärbungen des Plasmas keine Berücksichtigung finden.

Aus der US-3900396 ist bekannt, daß arterielles Blut Absorptionsmaxima bei Wellenlängen von 415, 550 und 593 nm aufweist. Diese Wellenlängen sind daher zur Untersuchung von Blutpräparaten besonders geeignet.

### Technische Aufgabe:

Der Erfindung liegt die Aufgabe zugrunde, ein Verfahren und eine Vorrichtung zum Fließtrennen von Vollblut als Gemisch von Flüssigkeiten in einzelne verschiedenfarbige Blutbestandteile, insbesondere zur Separation von Thrombozytenkonzentrat aus Buffycoat, zu schaffen, wobei der Trennvorgang selbsttätig gesteuert automatisch abläuft und insbesondere eine Trennung hoher Reinheit der einzelnen verschiedenfarbigen Bestandteile des Vollblutes erfolgt.

### Offenbarung der Erfindung und deren Vorteile:

Die Lösung der Aufgabe besteht in einem Verfahren zum Fließtrennen von Vollblut als Gemisch von Flüssigkeiten in einzelne verschiedenfarbige Blutbestandteile, unter Verwendung einer im Bereich einer lichttransparenten Verbindung angeordneten optisch-elektrischen Fotometereinheit, in der sich ein Sender und ein Empfänger für elektromagnetische Wellen befinden und an denen die Flüssigkeit innerhalb einer lichttransparenten Verbindung vorbeifließt, wobei als Sender der Fotometereinheit eine Lichtquelle mit einer mehr oder weniger monochromatischen Strahlung der Wellenlänge im Bereich entweder von cirka 535 nm bis 575 nm (grün bis rot), vorzugsweise 565 nm, oder von cirka 400 nm bis 453 nm (blau) verwendet wird, wobei das Licht vor dem Empfänger abgeblendet wird, so daß im wesentlichen nur parallele Lichtstrahlen auf dem Empfänger fallen, und der Empfänger ein auf die Wellenlänge abgestimmter Fotowiderstand oder eine Fotodiode oder ein Fototransistor ist, wobei vom Ausgangssignal des Empfängers automatisch das der Plasmaeigenfärbung entsprechende Signal abgezogen wird und das Differenzsignal zur Steuerung einer Unterbrechereinheit dient, durch die die Verbindung zwischen den Behältnissen geführt wird und die die Durchflußmenge regelt, insbesondere den Durchfluß bei Erreichen eines vorgegebenen Grenzwertes unterbricht.

Zur Erhöhung der Reinheit der abgepreßten Blutkomponente wird vom Ausgangssignal des Empfängers neben dem der Plasmaeigenfärbung entsprechenden Signal ein weiteres Signal geringer Amplitude abgezogen, wobei das Differenzsignal dann zur Steuerung des Durchflusses der Unterbrechereinheit dient.

Das Verfahren besitzt den Vorteil, daß damit eine vollautomatisch gesteuerte Separation des Vollblutes oder des Buffycoats in die einzelnen verschiedenfarbigen Bestandteile mittels fotometrischer Unterscheidung und Trennung der verschiedenfarbigen Bestandteile erfolgt und die einzelnen Bestandteile in hoher Reinheit voneinander getrennt und gewonnen werden können. Unabhängig von dem subjektiven Eindruck der beobachtenden Bedienungsperson wird ein vorgegebener Wert der Farberkennung eines Blutbestandteils von der Fotometereinheit immer wieder reproduziert. Der vorherige Abgleich erfolgt automatisch anhand der Plasmaeigenfärbung. Dabei kann ein maximaler unterer Grenzwert vorgegeben werden, wodurch lipämische und hämolytische Präparate automatisch von der Verarbeitung ausgeschlossen sind.

Der Erfinder hat erkannt, daß bei einer Spektralanalyse eines normalen Serums mit geringer Erythrozytenverunreinignng wie eines H₂O-Destillats mit lysierten Erythrozyten wie auch eines stark lipämischen Serums mit großer Erythrozytenverunreinigung sämtliche Flüssigkeiten im Bereich von 400 nm bis 453 nm (blau) und im Bereich von cirka 535 nm bis 575 nm (grün bis rot), vorzugsweise 565 nm, ausgeprägte (Hämoglobin-) Maxima im Blau- und im Grünbereich zeigen, nach denen deshalb die Detektion erfolgt.

Anstatt die Korrektur des Ausgangssignals einer Fotometereinheit auf die Plasmaeigenfärbung durchzuführen, können statt einer optisch-elektrischer Fotometereinheit der oben genannten Art zwei derartige Fotometereinheiten verwendet werden, die voneinander beabstandet sind. Dabei steuert die Differenz ΔV der Ausgangssignale der Empfänger den Durchfluß der Unterbrechereinheit, durch die die Schlauchverbindung zwischen den Behältnissen geführt wird.

Das Verfahren eignet sich gleichermaßen zur Detektion von Thrombozyten und/oder Leukozyten, weil diese aufgrund ihres Streueffekts detektiert werden können. Das die Verbindung durchquerende Licht, vorzugsweise kurzer Wellenlänge (blau oder grün-gelb bis rot), wird vor dem Empfänger abgeblendet, so daß im wesentlichen nur parallele Lichtstrahlen auf den Empfänger fallen können, wobei aufgrund Streuung des Lichts an den Thrombozyten und/oder Leukozyten wenig Lichtausbeute einen hohen Anteil an Thrombozyten und/oder Leukozyten in der Flüssigkeit bedeuten und umgekehrt.

Vorteilhaft kann zur Vermeidung eines ruckartigen Einfließens der Flüssigkeit in die Fotometereinheit die Durchflußmenge der Flüssigkeit in der Anfangsphase gedrosselt und der Durchflußquerschnitt zeitverzögert sehr langsam, insbesondere mechanisch-exzentrisch mittels eines mechanischen Exzenters, freigegeben werden. Wenn der Beutel mit Buffycoat in eine Abpreßeinheit des Standes der Technik gelegt wurde, so schoß in der Anfangsphase, weil Luft im Schlauch war, das Plasma bis zum Detektor. Erst wenn die Luft entwichen war, lief das Plasma langsam weiter. Dem konnte nur entgegengewirkt werden, in dem die Bedienungsperson am Anfang mit einer Klemmschere manuell entlüftete, was jedoch keine langsam-gleichmäßige Öffnung bewirkte.

Ebenso kann der Sender der Fotometereinheit in getakteter Weise angesteuert werden, wodurch die Fotometereinheit stabiler wird.

Eine erfindungsgemäße Vorrichtung zur Durchführung des Verfahrens besteht darin, daß der Sender der Fotometereinheit eine Lichtquelle, wie Sendediode oder Leuchtdiode (LED) ist mit einer mehr oder weniger monochromatischen Strahlung der Wellenlänge im Bereich entweder von cirka 535 nm bis 575 nm (grün bis rot), vorzugsweise 565 nm, oder von cirka 400 nm bis 453 nm (blau), und der Empfänger ein auf die Wellenlänge abgestimmter Fotowiderstand oder Fotodiode oder Fototransistor ist, wobei vor dem Empfänger eine Blende angeordnet ist, so daß im wesentlichen nur parallele Lichtstrahlen auf dem Empfänger fallen, und vom Ausgangssignal des Empfängers automatisch das der Plasmaeigenfärbung entsprechende Signal abgezogen wird und das Differenzsignal einer Unterbrechereinheit zugeführt wird, durch die die Verbindung zwischen den Behältnissen geführt wird, welche die Durchflußmenge in Abhängigkeit des Differenzsignals regelt, insbesondere den Durchfluß bei Erreichen eines vorgegebenen Grenzwertes unterbricht.

Die Vorrichtung ist um ein Vielfaches empfindlicher als das menschliche Auge; sie kann Thrombozyten und/oder Leukozyten sowie schon kleinste rote Erythrozytenmengen erkennen, was bedeutet, daß beim Abpreßvorgang des Buffycoats keine Erythrozyten oder Leukozyten mehr in den Thrombozytenbeutel bzw. in das Thrombozytenkonzentrat gelangen, weil die Vorrichtung vorher den Verbindungsschlauch abklemmt. Desweiteren wird eine gleichmäßige Strömungsgeschwindigkeit eingehalten. Die Verwirbelung des thrombozytenreichen Plasmas in der Buffycoatschicht mit der Leukozyten-Erythrozytenschicht tritt nicht auf (Siphoneffekt), weshalb keine Verunreinigung des Thrombozytenkonzentrates gegeben ist. Damit kann eine maximale thrombozytenreiche Plasmaausbeute erreicht werden. Desweiteren nimmt die Vorrichtung bei einer bestimmten Plasmaverunreinigung den Trennvorgang des Buffycoats nicht vor, es erfolgt eine sofortige Unterbrechung des Durchflusses mittels der Unterbrechereinheit. Desweiteren können praktisch sämtliche auf dem Markt befindlichen Abpreßeinheiten mit der Vorrichtung nachgerüstet werden.

In vorteilhafter Weise dient die Durchflußbegrenzungseinheit, beispielsweise ein Keil, ebenfalls zur Anpassung der Vorrichtung an den unterschiedlichen Preßdruck von unterschiedlichen handelsüblichen Preßeinheiten zum Abpressen derartiger Blutbeutel.

Ein weiterer Vorteil der Vorrichtung besteht darin, daß die Leukozyten- und Erythrozytenkonzentration im Thrombozytenkonzentrat deutlich geringer als beim Stand der Technik bzw. bei der manuellen Abpackmethode ist. Dies bedeutet eine medizinische Verbesserung des Thrombozytenkonzentrats, nämlich eine Verringerung der Alloimmunisierungsrate, was zu einer Reduzierung des Bedarfs an gefilterten bzw. Einspenderpräparaten führt. Ebenso ist ein gleichmäßigeres Füllgewicht von Thrombozytenplasma gegeben. Weiterhin ist auch eine Konzentration von Thrombozyten > 0,5x10¹¹ im Thrombozytenkonzentrat gewährleistet, welches mit der Vorrichtung gewonnen worden ist.

### Kurzbeschreibung der Zeichnung, in der zeigen:

- Figur 1: eine Trennstation bestehend aus einer Abpreßeinheit, einer Meßeinrichtung zur Unterscheidung des Plasma-Thrombozytenkonzentrats von den Erythrozyten- und Leukozytenzellen und einer Unterbrechereinheit zum Unterbrechen des Durchflusses zwischen zwei Beuteln
- Figur 2: ein Kompaktgerät mit einer Wiegeeinrichtung für den Beutel zur Gewinnung des Plasma-Thrombozytenkonzentrats zur Steuerung der Durchflußmenge innerhalb der Fotometereinheit
- Figur 3: ein Blockbild der wesentlichen Komponenten der Vorrichtung
- Figur 4: eine Draufsicht auf den Klemmblock der Figur 1
- Figur 5: eine Seitenansicht des Klemmblocks gemäß Figur 4
- Figur 6: eine um 90 Grad gedrehte Ansicht der Figur 5
- Figur 7: eine Ansicht der Unterseite des Klemmblocks
- Figur 8: ein Abdeckplättchen zum Abdecken des Klemmblocks
- Figur 9a, b, c: drei verschiedene Ansichten des Keils innerhalb des Klemmblocks zur Begrenzung des Durchflusses
- Figur 10 a, b, c: Spektralanalysen von H₂O-Destillat mit lysierten Erythrozyten, stark lipämischem Serum mit großer Erythrozytenverunreinigung normalem Serum mit geringer Erythrozytenverunreinigung
- Figur 11: die Vorderansicht einer weiteren Abpreßeinheit mit Öffnungsexzenter
- Figur 12: die Draufsicht auf die Abpreßeinheit der Figur 11 und
- Figur 13: ein Spannungs-Zeitdiagramm eines Thrombozytenkonzentrates

### Wege zur Ausführung der Erfindung:

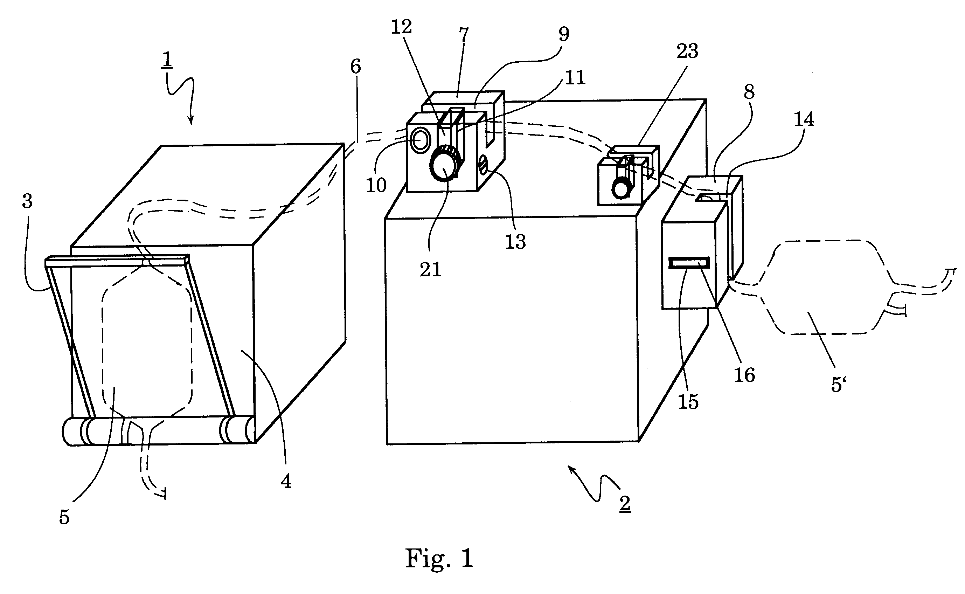

Figur 1 zeigt eine Vorrichtung zur automatisch gesteuerten Separation von Vollblut in die einzelnen Blutbestandteile bzw. zur Gewinnung eines thrombozytenreichen Plasmas, Thrombozytenkonzentrat, aus dem Buffycoat. Eine handelsübliche Abpreßeinheit 1, die eine solche für Vollblut oder für Buffycoat sein kann, besitzt in bekannter Weise an der Vorderseite 4 eine schwenkbar angeordnete Andruckplatte 3, die vorzugsweise durchsichtig ist, zwischen der und der Vorderseite 4 ein mit Vollblut oder Buffycoat gefüllter Beutel 5 plaziert ist. Der Beutel 5 ist über wenigstens einen lichtdurchlässigen flexiblen Schlauch 6 mit wenigstens einem Satellitenbeutel 5' verbunden zur Aufnahme der während der Separation gewonnenen verschiedenen Bestandteile des Blutes, in diesem Fall zur Gewinnung des Thrombozytenkonzentrats. Der Schlauch 6 ist über einen Klemmblock 7 geführt, der auf eine Meßeinrichtung 2 montiert ist, anschließend ist der Schlauch 6 durch eine Unterbrechereinheit 8 geführt.

Der Klemmblock 7 ist vorzugsweise lösbar auf der Meßeinrichtung 2 montiert, damit die Entfernung des Klemmblocks 7 von der Abpreßeinheit 1 und damit die Schlauchlänge des Schlauches 6 von der Abpreßeinheit 1 bis zum Klemmblock 7 vom Anwender variiert werden kann. Deshalb kann der Klemmblock 7 vorzugsweise eine eigene Einheit ohne einem Support darstellen, die nur mit ihren notwendigen Zuleitungen einschließlich des Schlauches 6 verbunden ist. Ebenso kann der Klemmblock jeweils auf die verschiedenen Abpreßeinheiten nachträglich montiert werden. Der Klemmblock 7, der in den Figuren 4 bis 7 gezeigt ist, ist vorzugsweise quaderförmig gestaltet und weist einen Längskanal 9 auf zum Einlegen des Schlauches 6 gemäß Figur 1. Quer zum Längskanal 9 ist innerhalb des Klemmblocks 7 ein Querkanal 11 angeordnet, in welchem sich als Durchflußbegrenzungseinheit ein Keil 12 verschieblich befindet, der in einer einfachen Version mittels einer Feststellschraube 13 in beliebiger vorgegebener Stellung innerhalb des Querkanals 11 arretiert werden kann. Die Verstellung des Keils 12 geschieht manuell vorzugsweise mit einer Verstellschraube 21, wie Mikrometerschraube, die in geeigneter Weise am Keil 12 angreift, um denselben bei Drehung transversal zu verschieben. Mittels des Keils 12 kann der in den Längskanal 9 eingelegte flexible Schlauch 6 mehr oder weniger abgeklemmt werden, um den Durchfluß durch den Schlauch 6 zu verändern.

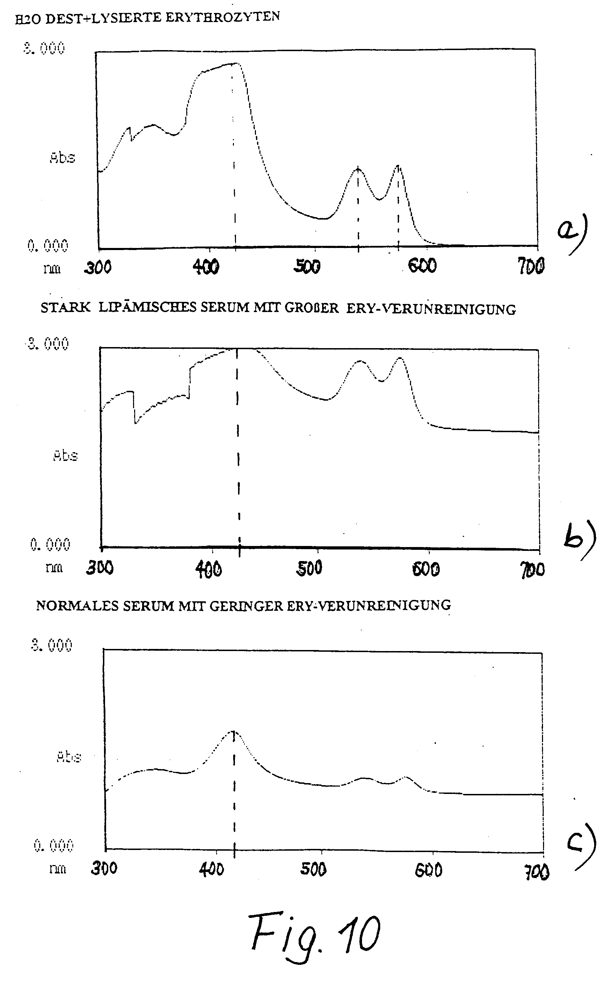

Der Klemmblock 7 beherbergt gleichzeitig eine Fotometereinrichtung, bestehend aus einem Sender 10, der eine Lichtquelle, insbesondere Leuchtdiode aufweist, und aus einem Empfänger 18, der ein Fotowiderstand oder eine Fotodiode oder ein Fototransistor ist. Sender 10 und Empfänger 18 sind vorzugsweise je in einer Bohrung quer zum Längskanal 9 des Klemmblocks 7 sich gegenüberstehend in Fließrichtung der Flüssigkeit vor dem Keil 12 angeordnet, was am besten aus Figur 4 zu ersehen ist. Die Lichtquelle 10 erzeugt eine vorzugsweise monochromatische Strahlung mit einer Wellenlänge von vorzugsweise 565 nm (grün), der Empfänger 18 besitzt seine größte Empfindlichkeit bzw. das größte Ausgangssignal bei der Durchleuchtung der roten Erythrozyten vorzugsweise im grünen Spektrum. Dabei kann der Wert der Thrombozyten-Leukozyten-Erythrozyten-Erkennung in weiten Grenzen vorgegeben werden. Mittels des Keils 12 wird die Fließgeschwindigkeit des Blutes durch den Klemmblock 7 sowie auch die Anpassung an verschiedene Abpreßeinrichtungen eingestellt. Auch über die entsprechende Schlauchlänge vor dem Fotometerkopf kann die Qualität des Thrombozytenkonzentrates noch verändert bzw. gesteuert werden. Um Fremdlichteinflüsse weitestgehend auszuschalten, ist der Klemmblock 7 mittels eines Abdeckplättchen abgedeckt, welches in Figur 8 gezeigt ist.

Desweiteren ist am Meßgerät 2 ein schematisch dargestellter Unterbrecherblock 8 angeordnet, der ebenfalls einen Kanal 14 aufweist, durch den der Schlauch 6 geführt ist. Zu diesem Kanal 14 ist geeignet ein Querkanal 15 angeordnet, in welchem ein Elektromagnet mit einem beweglichen Teil 16, der vorn in seinem Quetschbereich konisch zugespitzt ist, verschieblich angeordnet ist, und der mittels einer (nichtgezeigten) elektrischen Erregerspule innerhalb des Unterbrecherblocks 8 bewegt wird.

### Die Funktion der Vorrichtung ist folgende:

Der Buffycoat wird zwischen Andruckplatte 3 und vordere Gehäusewand 4 der Abpreßeinheit 1 gelegt; gleichzeitig wird der Verbindungsschlauch 6 in den Längskanal 9 des Klemmblocks 7 eingelegt. Das andere Ende des Schlauches 6 mit dem daran befestigten Satellitenbeutel 5' wird in die Unterbrechereinhheit 8 eingeführt. Nunmehr wird das Plasma durch die Andruckplatte 3 aus dem Beutel 5 herausgedrückt und in den Satellitenbeutel 5' überführt. Sobald die ersten Thrombozyten und/oder Leukozyten bzw. Erythrozyten im Schlauch 6 den Fotometerkopf passieren, d.h. sobald der eingestellte Wert der Erkennung innerhalb des Fotometers erreicht ist, gibt der Empfänger 18 einen Ausgangsimpuls ab, aufgrund desselben der bewegliche Teil 16 des Elektromagneten anspricht und den Schlauch 6 schlagartig abquetscht und dadurch die Unterbrechereinheit 8 den Zufluß in den Satellitenbeutel 5' unterbricht. Anschließend wird der Schlauch 6 durchtrennt, der Thrombozytenbeutel 5' entlüftet und das Schlauchende daran verschweißt.

Von entscheidender Bedeutung ist das Zusammenspiel zwischen dem Keil 12 und dem Fotometer innerhalb des Klemmblocks 7, weil nur durch die Einstellung einer gleichmäßigen, angepaßten Durchflußgeschwindigkeit mittels des Keils 12 Verunreinigungen des Thrombozytenkonzentrats durch die Buffycoatschicht wirkungsvoll vermieden werden. Das liegt daran, daß die erheblich größeren Leukozyten- und Erythrozytenzellen an der Verengung des Klemmblocks 7 aufgehalten und gestaut werden, hingegen die viel kleineren Thrombozytenplättchen die Verengung gemäß dem Stauwehrprinzip leicht passieren können. Falls die Durchflußgeschwindigkeit zu groß gewählt wird, werden schlagartig die größeren Leukozyten- und Erythrozytenzellen nunmehr mitgerissen. Der Keil 12 ist in verschiedenen Darstellungen in den Figuren 9 a, b und c dargestellt.

Es ist vorteilhaft, den Sender 10 in getakteter Weise anzusteuern, weil dadurch eine größere Lebensdauer und bessere Stabilisierung des Empfängers 18 erreicht werden. Ebenso kann der Sender bzw. die Sendediode eine eigene Stromversorgung besitzen. Bei der Einstellung des Empfängers 18 bzw. der Empfängerdiode werden zwei elektrische Schwellwerte vorgegeben, bei denen ein Schaltvorgang erfolgt; ebenso kann der Empfänger 18 eine eigene Stromversorgung aufweisen. Sobald der Fotometerkopf bzw. der Empfänger 18 ein Schließsignal an den Elektromagneten 16 gegeben hat, kann erst wieder eine Schaltung ausgelöst werden, wenn ein bestimmter Schwellwert erreicht worden ist.

Desweiteren kann eine Leuchtdiode zur Anwendung gelangen, die sowohl eine grüne und/oder bis rote und/oder blaue Strahlung spektral bzw. monochromatisch aussendet; der Empfänger bzw. die Empfangsdiode muß die unterschiedlichen Wellenlängen detektieren können. Auf diese Weise kann neben den roten Erythrozyten auch festgestellt werden, ob weitere dunkle oder andersfarbige Bestandteile, die nicht rot sind, sich innerhalb des Gemisches befinden. Auch in diesem Fall kann ein Unterbrechungsvorgang des Durchflusses ausgelöst werden. Nach dem Auslösen des Elektromagneten der Unterbrechereinheit 8 muß der Elektromagnet unbedingt angezogen bleiben, bis die Bedienungsperson ihn freigibt.

Die Fotometereinheit erfaßt nicht nur Erythrozyten, die farblich zum Gelb-Rotbereich tendieren und vorzugsweise mittels Licht der Wellenlänge 565 nm erfaßt werden, sondern auch Thrombozyten und Leukozyten, die ja mehr oder weniger weiß sind und mit dem bloßen Auge und aufgrund ihrer Kleinheit im Plasma nicht erkannt werden können. Es ist zur Herstellung von hochreinem Thrombozytenkonzentrat besonders wichtig, diese zu detektieren. Aufgrund des kurzwelligen Lichts (blau oder grün bis rot) erfolgt durch die kleinen Thrombozyten und auch durch die Leukozyten ein Streueffekt, der ausgewertet werden kann, indem vor dem Empfänger der Fotometereinheit eine verstellbare Blende vorgesehen und diese so klein gewählt wird, daß praktisch nur paralleles Licht am Empfänger eintrifft. Je mehr Thrombozyten und/oder Leukozyten in der Flüssigkeit vorhanden sind, umso weniger Licht kommt am Empfänger an, weil das gestreute Licht die Blende nicht passieren kann. Sind keine Thrombozyten und/oder Leukozyten vorhanden, so erfolgt wenig Streuung, so daß damit eine große Lichtausbeute am Empfänger des Fotometers verbunden ist, wobei durch Leukozyten allein eine noch größere Streuung erfolgt.

Wie oben ausgeführt, kann zur Vermeidung eines ruckartigen Einfließens der Flüssigkeit in die Fotometereinheit die Durchflußmenge in der Anfangsphase gedrosselt und der Durchflußquerschnitt zeitverzögert sehr langsam, insbesondere mechanisch-exzentrisch mittels eines mechanischen Exzenters, freigegeben werden. Dazu kann der Schlauch 6 durch einen separaten mechanischen Öffnungsexzenter 23 geführt sein, der in unmittelbarer Nähe der Unterbrechereinheit 8 angeordnet ist. Oder der bewegliche Teil 16 des Elektromagneten der Unterbrechereinheit 8 kann zusätzlich als Öffnungsexzenter 23 wirken und in Unterbrechereinheit 8 integriert sein.

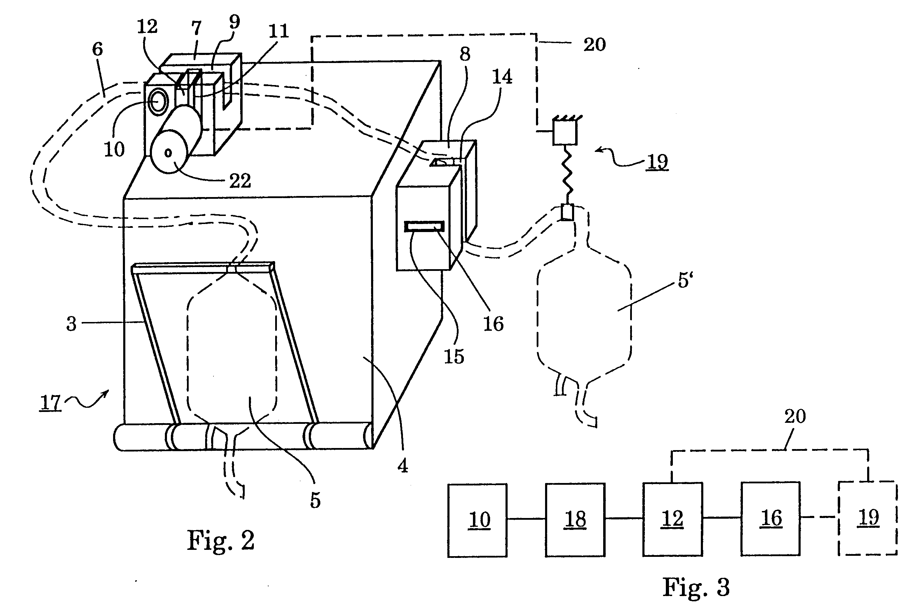

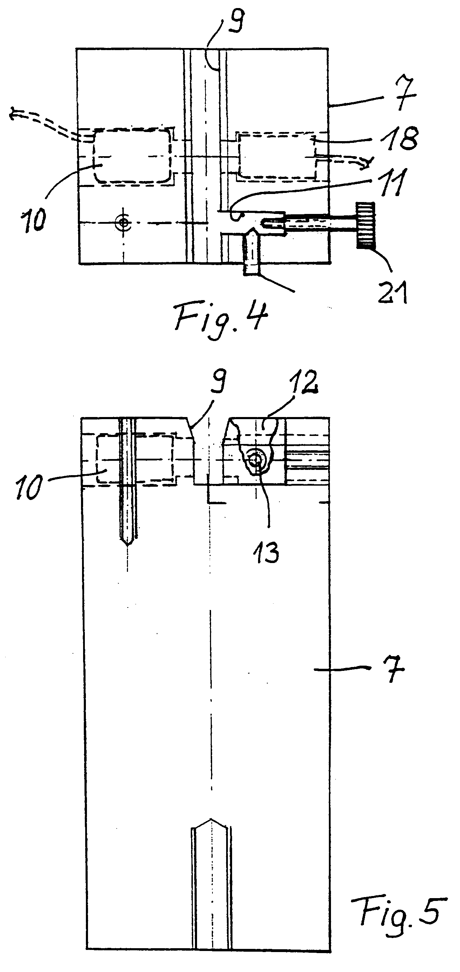

Figur 2 zeigt ein weiteres Beispiel eines Separator-Kompaktgerätes 17, wobei gleiche Teile wie in Figur 1 mit gleichen Bezugsziffern bezeichnet sind.

Im Unterschied zu Figur 1 kann hier noch die Fließgeschwindigkeit des Thrombozytenkonzentrats durch den Schlauch 6 in den Satellitenbeutel 5' geregelt werden. Dazu hängt der Satellitenbeutel 5' an einem Wiegesystem 19, welches in Abhängigkeit des Gewichts des Beutels 5' fortlaufend ein elektrisches Stellsignal erzeugt, das über eine Leitung 20 auf einen Elektromotor 22 oder Schrittmotor innerhalb des Klemmblocks 7 gegeben wird. Der Elektromotor verstellt motorisch den Keil 12 innerhalb des Querkanals 11 transversal, so daß der Schlauch mehr oder weniger abgequetscht wird und der Fließwiderstand der Flüssigkeit durch den Schlauch 6 und somit der Durchfluß geregelt werden kann. Über die Gewichtszunahme des Beutels 5' pro Minute wird die Fließgeschwindigkeit bestimmt und in Abhängigkeit der Schwellwerte des Fotometers entsprechend geregelt. Zusätzlich kann der Keil als Öffnungsexzenter wirken und über den Elektromotor 22 sehr langsam geöffnet werden.

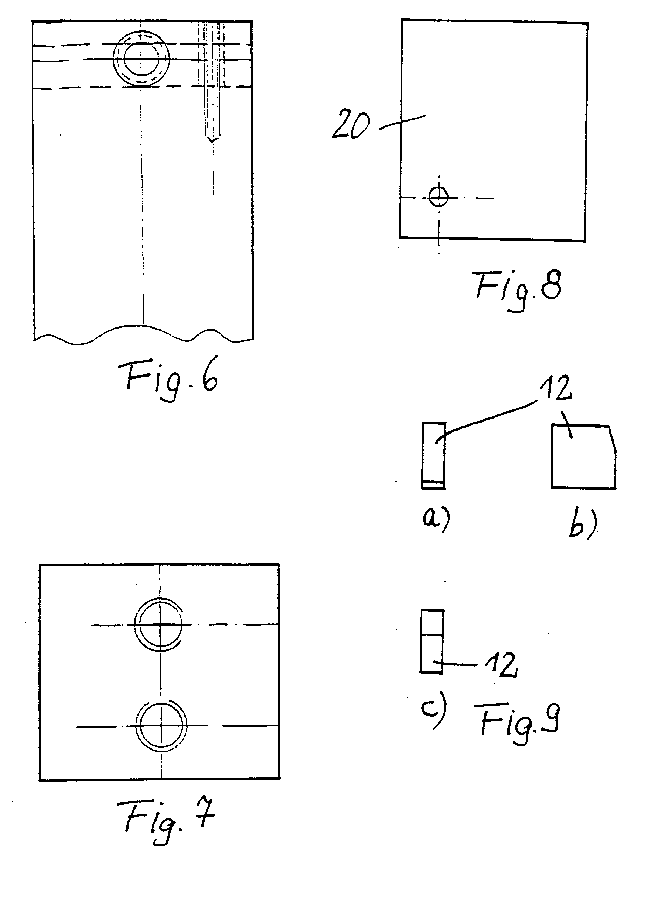

Figur 3 zeigt ein Blockbild der aufeinanderfolgenden wesentlichen Komponenten der Vorrichtung, bestehend aus Sender 10, Empfänger 18 und Keil 12, dem der Elektromagnet 16 nachgeschaltet ist. Die gestrichelt gezeichnete Wägeeinrichtung wirkt über die Leitung 20 zurück auf die Verstelleinrichtung zum Verstellen des Keils 12, die mittels eines Elektromotors 22 angetrieben werden kann. Der Sender der Fotometereinheit 10 kann ein solcher zur Abstrahlung sowohl von grüner und/oder roter und/oder blauer Strahlung sein; in diesem Fall besitzt der Empfänger 18 eine entsprechende Bandbreite und ist imstande, die unterschiedlichen Wellenlängen zu unterscheiden. Der Sender der Fotometereinheit kann eine Weißlichtlampe mit einem Filter sein zur Erzeugung einer grünen und/oder roten und/oder blauen monochromatischen Strahlung. In diesem Fall ist der Empfänger ebenfalls entsprechend breitbandig ausgelegt. Zum langsamen Öffnen des Durchlaßquerschnittes des Schlauches kann auch ein Exzenter auf den Keil 12 der Durchflußbegrenzungseinheit 7 einwirken, womit der Keil den Durchlaßquerschnitt sehr langsam freizugeben imstande ist. Oder der Schrittmotor kann den Keil exzentrisch ungleichmäßig verschieben. Bis das Plasma kommt, wird somit die Klemmung verstärkt, danach wird der Querschnitt langsam freigegeben.

Die Figur 10 a, b, c zeigt drei Spektralanalysen, nämlich a) von H₂O-Destillat mit lysierten Erythrozyten, b) von stark lipämischem Serum mit großer Erythrozytenverunreinigung sowie c) von normalem Serum mit geringer Erythrozytenverunreinigung. Man erkennt, daß in allen drei Fällen im Bereich entweder von cirka 535 nm bis 575 nm (grün, gelb bis rot), vorzugsweise 565 nm, sowie von cirka 400 nm bis 453 nm (blau) mehr oder weniger ausgeprägte (Hämoglobin-) Absorptions-Maxima vorhanden sind, die detektiert werden können.

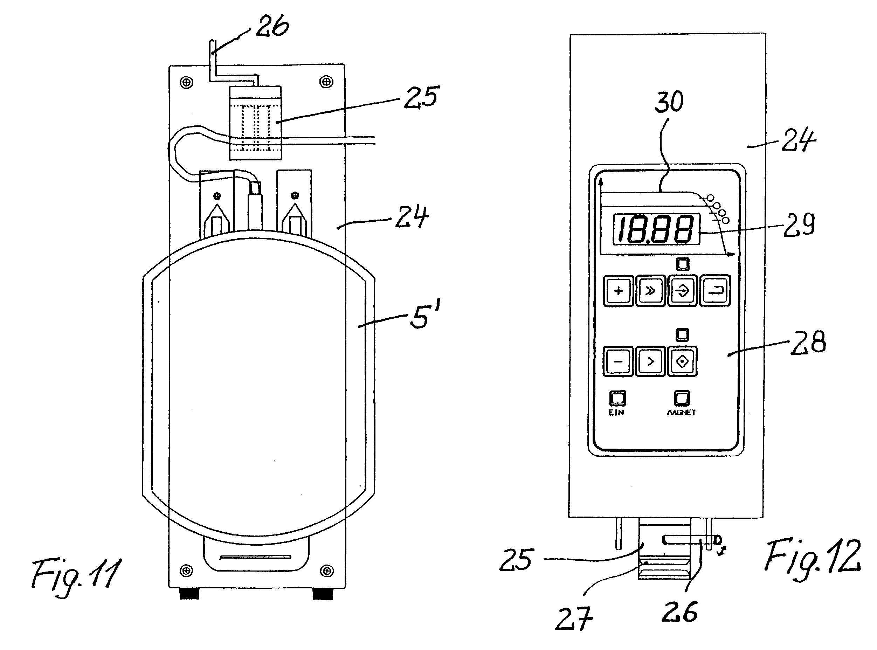

Die Figuren 11 und 12 zeigen eine weitere Meßeinrichtung 24 mit einem Beutel 5', oberhalb dem sich eine Unterbrechereinheit 25 befindet, durch die wie in den Figuren 1 oder 2 der Schlauch des Beutels gelegt ist und dessen beweglicher Teil des Elektromagneten zusätzlich als Öffnungsexzenter ausgebildet ist (die separate Fotometereinheit ist nicht gezeigt). Der bewegliche Teil des Magneten kann mittels einer kleinen Handkurbel 26 manuell geöffnet oder geschlossen werden. In der Draufsicht der Figur 12 auf die Meßeinrichtung 24 ist der bewegliche Teil mit einem beweglichen Klemmbacken 27 zu erkennen, dessen Bewegung beim Öffnungsvorgang durch die Handkurbel 26 anfangs ganz langsam erfolgt und der bei Betätigung durch den elektromagneten schlagsrtig schließt. Die Meßeinrichtung 24 besitzt desweiteren ein Display 28 mit Tastatur, über die die individuelle Einstellung erfolgt, und beinhaltet eine Spannungsanzeige 29, mittels der die momentane Färbung einschließlich Thrombozyten und Leukozyten in der Einheit [Volt] angezeigt wird.

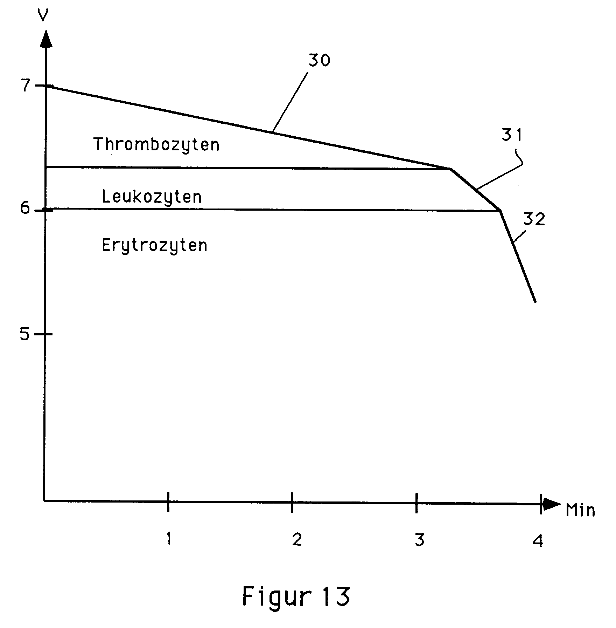

Figur 13 zeigt ein typisches Spannungs-Zeitdiagramm eines Thrombozytenkonzentrates. Während des Abziehens der Thrombozyten fällt die Spannung linear wenig ab, es ergibt sich der Ast 30. Sobald die Abfallkurve die Steigung des Astes 31 erreicht, was aufgrund des Abzugs von Leukozyten der Fall ist, wird abgeklemmt. Wenn Erythrozyten abgezogen werden, erfolgt nochmals ein massiver Spannungsabfall entsprechend dem Ast 32. Aus diesem Diagramm wird deutlich, daß es zur Gewinnung eines höchst reinen Thrombozytenkonzentrates wichtig ist, Thrombozyten und Leukozyten zu detektieren.

Um ein sehr reines Thrombozytenkonzentrat zu erhalten, muß von der individuellen Eigenfärbung des Plasmas ein geringer Spannungswert, zum Beispiel 0,75 V, abgezogen werden, was automatisch in einem Speicher der Vorrichtung erfolgt. Wenn der Wert erreicht ist, klemmt der Elektromagnet 16 der Unterbrechereinheit 8 ab, der Elektromagnet 16 öffnet nur dann, wenn ein bestimmter Wert erreicht wird. Durch den individuellen Abzug der Eigenfärbung des Plasmas wird erreicht, daß mindestens 90% des Plasmas abgepreßt wird bis auf ein Restvolumen, welches im Beutel in der Abpreßeinheit 1 verbleibt. Um den Rest ebenfalls zu gewinnen, kann nochmals gestartet werden, aber mit einem geringeren Spannungs-Abzugswert. Deshalb wird der momentane Spannungswert abgespeichert abzüglich eines anschließend geringeren Abzugswertes von beispielsweise 0,3 V. Derselbe Effekt kann durch die Einstellung eines ΔV erreicht werden, welches der Steigung des Astes 31 entspricht. Sobald vom Spannungsast 30 zum Spannungsast 31 übergegangen wird, kann gezielt abgeklemmt werden, was einer noch besseren Optimierung entspricht.

Es ist möglich, hierfür zwei Sensorsysteme, vorzugsweise mit einer Meßbrücke zum Abgleich, vorzusehen, die entweder innerhalb des Klemmblockes 7 ungefähr 1 cm voneinander entfernt sind oder sich das eine im Klemmblock 7, das andere in der Unterbrechereinheit 8 befindet. Damit kann eine differentielle Messung (ΔV) erzielt und eine genaue Abklemmung ermöglicht werden.

### Gewerbliche Anwendbarkeit:

Das Verfahren und die Vorrichtung sind dazu geeignet, Vollblut oder Buffycoat, welches sich nach einer bzw. zwei Zentrifugationen in einem Beutel mit Satellitenbeuteln befindet, die mittels durchsichtiger Schläuche miteinander verbunden sind, in seine verschiedenfarbige Bestandteile zu zerlegen, insbesondere zur Gewinnung eines hochreinen Thrombozytenkonzentrats. Der Abpreßvorgang des Beutels bzw. des Buffycoats wird mittels der Vorrichtung nach den verschiedenfarbigen Anteilen des Blutes automatisch gesteuert, wobei bei der Behandlung des Buffycoats die Bestandteile thrombozytenreiches Plasma, angereichert mit Thrombozyten einerseits und restlichen Erythrozyten und Leukozyten andererseits, gewonnen werden.

### Liste der Bezugszeichen:

- 1: Abpreßeinheit
- 2, 24: Meßeinrichtung
- 3, 27: Andruckplatte
- 4: Gehäusewand
- 5,5': Beutel
- 6: Schlauch
- 7: Klemmblock
- 8, 25: Unterbrechereinhheit
- 9,14: Kanäle
- 10: Sender bzw. Leuchtdiode
- 11,15: Querkanäle
- 12: Keil
- 13: Feststellschraube
- 16: Elektromagnet
- 17: Separator-Kompaktgerät
- 18: Empfänger
- 19: Wiegesystem
- 20: Abdeckplättchen
- 21: Verstellschraube
- 22: Elektromotor
- 23: Öffnungsexzenter
- 26: Handkurbel
- 27: Klemmbacken
- 28: Display
- 29: Spannungsanzeige
- 30, 31, 32: Kurvenäste

## Patentansprüche

1. Verfahren zum Fließtrennen von Vollblut als Gemisch von Flüssigkeiten in einzelne verschiedenfarbige Blutbestandteile, welches in flexible Behältnissen (5,5'), insbesondere Beutel, abgepackt ist, die untereinander durch eine wenigstens zum Teil lichttransparente Verbindung (6), insbesondere flexibler Schlauch, miteinander verbunden sind, und die Blutbestandteile bei Krafteinwirkung von einem Behältnis durch die lichttransparente Verbindung in ein anderes Behältnis fließen, insbesondere zur Separation von Thrombozytenkonzentrat aus Buffycoat, unter Verwendung einer im Bereich einer lichttransparenten Verbindung angeordneten optisch-elektrischen Fotometereinheit (10,18), in der sich ein Sender (10) und ein Empfänger (18) für elektromagnetische Wellen befinden und an denen die Flüssigkeit innerhalb der lichttransparenten Verbindung vorbeifließt, wobei als Sender (10) der Fotometereinheit (10,18) eine Lichtquelle, wie Sendediode (10) oder Leuchtdiode (LED), mit einer mehr oder weniger monochromatischen Strahlung der Wellenlänge im Bereich entweder von cirka 535 nm bis 575 nm (grün bis rot), vorzugsweise 565 nm, oder von cirka 400 nm bis 453 nm (blau) verwendet wird, dadurch gekennzeichnet,
daß das Licht vor dem Empfänger abgeblendet wird, so daß im wesentlichen nur parallele Lichtstrahlen auf dem Empfänger fallen, und der Empfänger (18) ein auf die Wellenlänge abgestimmter Fotowiderstand oder eine Fotodiode (18) oder ein Fototransistor ist, wobei vom Ausgangssignal des Empfängers (18) automatisch das der Plasmaeigenfärbung entsprechende Signal abgezogen wird und das Differenzsignal zur Steuerung einer Unterbrechereinheit (16) dient, durch die die Verbindung zwischen den Behältnissen geführt wird und die die Durchflußmenge regelt, insbesondere den Durchfluß bei Erreichen eines vorgegebenen Grenzwertes unterbricht.

2. Verfahren nach Anspruch 1, dadurch gekennzeichnet,
daß ein maximaler Wert für die Plasmaeigenfärbung vorgegeben und die Verarbeitung des Blutpräparats bei Überschreitung dieses Werts ausgeschlossen wird, wodurch lipämische und hämolytische Präparate automatisch von der Verarbeitung ausgeschlossen sind.

3. Verfahren nach Anspruch 1 oder 2, dadurch gekennzeichnet,
daß zur Erhöhung der Reinheit der abgepreßten Blutkomponente vom Ausgangssignal des Empfängers neben dem der Plasmaeigenfärbung entsprechenden Signal ein weiteres Signal geringer Amplitude abgezogen wird, wobei das Differenzsignal zur Steuerung des Durchflusses der Unterbrechereinheit (16) dient.

4. Verfahren zum Fließtrennen von Vollblut als Gemisch von Flüssigkeiten in einzelne verschiedenfarbige Blutbestandteile, welches in flexible Behältnissen (5,5'), insbesondere Beutel, abgepackt ist, die untereinander durch eine wenigstens zum Teil lichttransparente Verbindung (6), insbesondere flexibler Schlauch, miteinander verbunden sind, und die Blutbestandteile bei Krafteinwirkung von einem Behältnis durch die lichttransparente Verbindung in ein anderes Behältnis fließen, insbesondere zur Separation von Thrombozytenkonzentrat aus Buffycoat, unter Verwendung einer im Bereich einer lichttransparenten Verbindung angeordneten optisch-elektrischen Fotometereinheit (10,18), in der sich ein Sender (10) und ein Empfänger (18) für elektromagnetische Wellen befinden und an denen die Flüssigkeit innerhalb der lichttransparenten Verbindung vorbeifließt, wobei als Sender (10) der Fotometereinheit (10,18) eine Lichtquelle, wie Sendediode (10) oder Leuchtdiode (LED), mit einer mehr oder weniger monochromatischen Strahlung der Wellenlänge im Bereich entweder von cirka 535 nm bis 575 nm (grün bis rot), vorzugsweise 565 nm, oder von cirka 400 nm bis 453 nm (blau) dient, dadurch gekennzeichnet,
daß zwei voneinander beabstandete optisch-elektrische Fotometereinheiten (10,18) verwendet werden, wobei das Licht vor dem Empfänger abgeblendet wird, so daß im wesentlichen nur parallele Lichtstrahlen auf dem Empfänger fallen, und der Empfänger (18) ein auf die Wellenlänge abgestimmter Fotowiderstand oder eine Fotodiode (18) oder ein Fototransistor ist, und aus der Differenz ΔV der Ausgangssignale der Empfänger der Fotometereinheiten ein Signal abgeleitet wird, welches den Durchfluß einer Unterbrechereinheit (16) steuert, durch die die Verbindung zwischen den Behältnissen geführt wird.

5. Verfahren nach Anspruch 1 oder 4, dadurch gekennzeichnet,
daß zur Vermeidung eines ruckartigen Einfließens der Flüssigkeit in die Fotometereinheit (10,18) die Durchflußmenge der Flüssigkeit in der Anfangsphase gedrosselt und der Durchflußquerschnitt bis nach dem Entweichen von Luft zeitverzögert sehr langsam, insbesondere in mechanisch-exzentrischer Weise, freigegeben wird.

6. Verfahren nach Anspruch 1 oder 4, dadurch gekennzeichnet,
daß der Sender (10) der Fotometereinheit in getakteter Weise angesteuert wird.

7. Verfahren nach nach Anspruch 1 oder 4, dadurch gekennzeichnet,
daß zur Detektion von Thrombozyten und/oder Leukozyten das die Verbindung durchquerende Licht, vorzugsweise kurzer Wellenlänge, vor dem Empfänger (18) abgeblendet wird, so daß im wesentlichen nur parallele Lichtstrahlen auf den Empfänger (18) fallen, wobei aufgrund Streuung des Lichts an den Thrombozyten und/oder Leukozyten wenig Lichtausbeute einen hohen Anteil an Thrombozyten und/oder Leukozyten in der Flüssigkeit bedeuten und umgekehrt.

8. Vorrichtung zum Fließtrennen von Vollblut als Gemisch von Flüssigkeiten in einzelne verschiedenfarbige Blutbestandteile, welches in flexible Behältnissen (5,5'), insbesondere Beutel, abgepackt ist, die untereinander durch eine wenigstens zum Teil lichttransparente Verbindung (6), insbesondere flexibler Schlauch, miteinander verbunden sind, und die Blutbestandteile bei Krafteinwirkung von einem Behältnis durch die lichttransparente Verbindung in ein anderes Behältnis fließen, insbesondere zur Separation von Thrombozytenkonzentrat aus Buffycoat, mit einer optisch-elektrischen Fotometereinheit (10,18), in der sich ein Sender (10) und ein Empfänger (18) für elektromagnetische Wellen befinden und an denen die Flüssigkeit innerhalb der lichttransparenten Verbindung vorbeifließt, wobei der Sender der Fotometereinheit eine Lichtquelle (10), wie Sendediode (10) oder Leuchtdiode (LED) ist mit einer mehr oder weniger monochromatischen Strahlung der Wellenlänge im Bereich entweder von cirka 535 nm bis 575 nm (grün bis rot), vorzugsweise 565 nm, oder von cirka 400 nm bis 453 nm (blau), dadurch gekennzeichnet,
daß der Empfänger (18) ein auf die Wellenlänge abgestimmter Fotowiderstand oder Fotodiode (18) oder Fototransistor ist, wobei vor dem Empfänger eine Blende angeordnet ist, so daß im wesentlichen nur parallele Lichtstrahlen auf dem Empfänger fallen, und vom Ausgangssignal des Empfängers (18) automatisch das der Plasmaeigenfärbung entsprechende Signal abgezogen wird und das Differenzsignal einer Unterbrechereinheit (16) zugeführt wird, durch die die Verbindung zwischen den Behältnissen geführt wird, welche die Durchflußmenge in Abhängigkeit des Differenzsignals regelt, insbesondere den Durchfluß bei Erreichen eines vorgegebenen Grenzwertes unterbricht.

9. Vorrichtung zum Fließtrennen von Vollblut als Gemisch von Flüssigkeiten in einzelne verschiedenfarbige Blutbestandteile, welches in flexible Behältnissen (5,5'), insbesondere Beutel, abgepackt ist, die untereinander durch eine wenigstens zum Teil lichttransparente Verbindung (6), insbesondere flexibler Schlauch, miteinander verbunden sind, und die Blutbestandteile bei Krafteinwirkung von einem Behältnis durch die lichttransparente Verbindung in ein anderes Behältnis fließen, insbesondere zur Separation von Thrombozytenkonzentrat aus Buffycoat, mit einer optisch-elektrischen Fotometereinheit (10,18), in der sich ein Sender (10) und ein Empfänger (18) für elektromagnetische Wellen befinden und an denen die Flüssigkeit innerhalb der lichttransparenten Verbindung vorbeifließt, wobei der Sender der Fotometereinheit eine Lichtquelle (10), wie Sendediode (10) oder Leuchtdiode (LED) ist mit einer mehr oder weniger monochromatischen Strahlung der Wellenlänge im Bereich entweder von cirka 535 nm bis 575 nm (grün bis rot), vorzugsweise 565 nm, oder von cirka 400 nm bis 453 nm (blau), dadurch gekennzeichnet,
daß die Vorrichtung zwei optisch-elektrische Fotometereinheiten (10,18) aufweist, wobei vor deren Empfängern jeweils eine Blende angeordnet ist, so daß im wesentlichen nur parallele Lichtstrahlen auf den Empfänger fallen, und der Empfänger (18) ein auf die Wellenlänge abgestimmter Fotowiderstand oder Fotodiode (18) oder Fototransistor ist, und aus der Differenz ΔV der Ausgangssignale der Empfänger der Fotometereinheiten ein Signal abgeleitet wird, welches einer Unterbrechereinheit (16) zugeführt wird, durch die die Verbindung zwischen den Behältnissen geführt wird, welche die Durchflußmenge in Abhängigkeit des Differenzsignals regelt, insbesondere den Durchfluß bei Erreichen eines vorgegebenen Grenzwertes unterbricht.

10. Vorrichtung nach Anspruch 9, dadurch gekennzeichnet,
daß die Fotometereinheiten (10,18) mit einer Meßbrücke zum Abgleich versehen sind.

11. Vorrichtung nach Anspruch 8 oder 9, gekennzeichnet durch
einen Klemmblock (7), in den die Fotometereinheit integriert und innerhalb desselben die lichttransparente Verbindung (6) zwischen den Behältnissen (5,5') der Flüssigkeit geführt ist und Sender (10) und Empfänger (18) sich in Nachbarschaft zur Verbindung befinden, eine Durchflußbegrenzungseinheit (12) ebenfalls zur Begrenzung bzw. Regelung des Durchflusses der Flüssigkeit, die in Nachbarschaft nach der Fotometereinheit angeordnet ist und die einen einstellbaren Fließwiderstand für die Flüssigkeit innerhalb der Verbindung (6) bildet.

12. Vorrichtung nach Anspruch 8 oder 9, dadurch gekennzeichnet,
daß die Unterbrechereinheit zum Abklemmen des Verbindungsschlauches zwischen zwei Beuteln einen beweglich gehalterten Elektromagnet (16) aufweist, der vom Empfänger (18) der Fotometereinheit ansteuerbar ist.

13. Vorrichtung nach Anspruch 8 oder 9, dadurch gekennzeichnet,
daß zur Unterscheidung der unterschiedlichen Wellenlängen der Empfänger (18) eine entsprechende Bandbreite jeweils im Grün-Rotbereich bzw. Blaubereich besitzt, beispielsweise ± 15% des Wellenlängenbereiches.

14. Vorrichtung nach Anspruch 8 oder 9, dadurch gekennzeichnet,
daß der Sender der Fotometereinheit eine Weißlichtlampe mit einem Filter zur Erzeugung einer grünen und/oder roten und/oder blauen monochromatischen Strahlung ist.

15. Vorrichtung nach Anspruch 8 oder 9, dadurch gekennzeichnet,
daß der Klemmblock (7) einen Längskanal (9) aufweist, innerhalb desselben der Schlauch (6) geführt ist und in den ein Querkanal (11) mündet, innerhalb desselben die Durchflußbegrenzungseinheit (12) angeordnet ist.

16. Vorrichtung nach Anspruch 8 oder 9, dadurch gekennzeichnet,
daß die Durchflußbegrenzungseinheit ein Keil (12) ist, der verschieblich innerhalb des Querkanals (11) angeordnet ist zum geringeren oder stärkeren Zusammenquetschen des Schlauches (6).

17. Vorrichtung nach Anspruch 8 oder 9, dadurch gekennzeichnet,
daß der Keil (12) manuell mittels einer Schraube (21), wie Mikrometerschraube (21), oder motorisch mittels eines Elektromotors, insbesondere Schrittmotor, und einer mit dem Keil und dem Elektromotor gekoppelten Verschiebeeinrichtung verschieblich ist.

18. Vorrichtungnach Anspruch 8 oder 9, dadurch gekennzeichnet,
daß der zweite Beutel (5'), in welchen ein Teil des Gemisches aus dem ersten Beutel (5) abgedrückt wird, an einer Wägeeinrichtung (19) aufgehängt ist zur fortlaufenden Feststellung des Füllgewichts des Beutels.

19. Vorrichtung nach Anspruch 18, dadurch gekennzeichnet,
daß die Wägeeinrichtung einen elektrischen Stellwertgeber aufweist, der ein elektrisches Regelsignal entsprechend dem sich ändernden Gewicht des Beutels (5') auf den Elektromotor abgibt, der am Klemmblock (7) befestigt ist und auf die Verschiebeeinrichtung einwirkt zum geregelten Vor-und-Zurück-Verschieben des Keils (12) innerhalb des Querkanals (11) des Klemmblocks (7).

20. Vorrichtung nach Anspruch 8 oder 9, dadurch gekennzeichnet,
daß zugeordnet zur Unterbrechereinheit (8) als Abklemmeinheit für den Schlauch (6) ein mechanischer Öffnungsexzenter (23) angeordnet ist, der bei Betätigung den Durchlaßquerschnitt des Schlauches (6) sehr langsam öffnet.

21. Vorrichtung nach Anspruch 20, dadurch gekennzeichnet,
daß die Abklemmeinheit in die Unterbrechereinheit (8) integriert ist und der bewegliche Teil des Elektromagneten (16) sehr langsam zu öffnen imstande ist.

22. Vorrichtung nach Anspruch 21, dadurch gekennzeichnet,
daß die Abklemmeinheit in die Durchflußbegrenzungseinheit (7) integriert ist und der Keil (12) zusätzlich als Öffnungsexzenter mittels des Elektromotors (22) beweglich ist.

## Claims

1. Process for the fluid separation of whole blood as a mixture of fluids into individual, differently-colored blood constituents, the whole blood being packaged in flexible containers (5, 5'), especially bags, which are connected to each other by a connection (6) that is at least partially translucent, especially a flexible tube and, when force is applied, the blood constituents flow from one container through the translucent connection into another container, especially for the separation of a thrombocyte concentrate from buffy coat, using an optical-electric photometer unit (10, 18) that is positioned in the area of a translucent connection and that comprises a transmitter (10) and a receiver (18) for electromagnetic waves and past which tile fluid flows inside the translucent connection, whereby a light source such as a transmitting diode (10) or a light-emitting diode (LED) with a more or less monochromatic light having a wavelength within the range either from about 535 nm to 575 nm (green to red), preferably 565 nm, or else about 400 nm to 453 nm (blue) is used as the transmitter (10) of the photometer unit (10,18), characterized in that
the light is stopped down before the receiver so that essentially only parallel beams of light strike the receiver, and the receiver (18) is a photoresistor or a photodiode (18) or a phototransistor adapted to the wavelength, whereby the signal correspond-responding to the inherent color of the plasma is automatically subtracted from the output signal of the receiver (18), and the differential signal serves to control an interrupter unit (16) through which the connection between the containers passes and which regulates the flow rate, in particular interrupting the flow when a predefined limit value is reached.

2. Process according to Claim 1, characterized in that
a maximum value for the inherent color of the plasma is specified and the processing of any blood preparation is precluded when this value is exceeded, as a result of which lipidic and hemolytic preparations are automatically excluded from the processing.

3. Process according to Claim 1 or 2, characterized in that,
in order to increase the purity of the blood constituent being squeezed out, in addition to the signal corresponding to the inherent color of the plasma, another signal of a smaller amplitude is subtracted from the output signal of the receiver, whereby the differential signal then serves to control the flow of the interrupter unit (16).

4. Process for the fluid separation of whole blood as a mixture of fluids into individual, differently-colored blood constituents, the whole blood being packaged in flexible containers (5, 5'), especially bags, which are connected to each other by a connection (6) that is at least partially translucent, especially a flexible tube and, when force is applied, the blood constituents flow from one container through the translucent connection into another container, especially for the separation of a thrombocyte concentrate from buffy coat, using an optical-electric photometer unit (10, 18) that is positioned in the area of a translucent connection and that comprises a transmitter (10) and a receiver (18) for electromagnetic waves and past which the fluid flows inside the translucent connection, whereby a light source such as a transmitting diode (10) or a light-emitting diode (LED) with a more or less monochromatic light having a wavelength within the range either from about 535 nm to 575 nm (green to red), preferably 565 nm, or else about 400 nm to 453 nm (blue) serves as the transmitter (10) of the photometer unit (10,18),
characterized in that
two optical-electric photometer units (10, 18) at a distance from each other are used, whereby the light is stopped down before the receiver so that essentially only parallel beams of light strike the receiver, and the receiver (18) is a photoresistor or a photodiode (18) or a phototransistor adapted to the wavelength and, from the difference ΔV of the output signals of the receivers of the photometer units, a signal is derived that controls the flow of an interrupter unit (16) through which the connection between the containers passes.

5. Process according to Claim 1 or 4, characterized in that,
in order to prevent the fluid from flowing abruptly into the photometer unit (10,18), the flow quantity of the fluid can be throttled in the initial phase and the flow cross section can be opened very slowly with a time delay until after air escapes, especially in a mechanical-eccentric manner.

6. Process according to Claim 1 or 4, characterized in that
the transmitter (10) of the photometer unit is regulated in a switched mode.

7. Process according to Claim 1 or 4, characterized in that,
in order to detect thrombocytes and/or leukocytes, the light that passes through the connection, preferably having a short wavelength, is stopped down before the receiver (18) so that essentially only parallel beams of light see the receiver (18); due to the scatter of the light on the thrombocytes and/or leukocytes, a low light yield means a high proportion of thrombocytes and/or leukocytes in the fluid and vice versa.

8. Device for the fluid separation of whole blood as a mixture of fluids into individual, differently-colored blood constituents, the whole blood being packaged in flexible containers (5, 5'), especially bags, which are connected to each other by a connection (6) that is at least partially translucent, especially a flexible tube and, when force is applied, the blood constituents flow from one container through the translucent connection into another container, especially for the separation of a thrombocyte concentrate from buffy coat, with an optical-electric photometer unit (10, 18) that comprises a transmitter (10) and a receiver (18) for electromagnetic waves and past which the fluid flows inside the translucent connection, whereby the transmitter (10) of the photometer unit (10,18) is a light source such as a transmitting diode (10) or a light-emitting diode (LED) with a more or less monochromatic light having a wavelength within the range either from about 535 nm to 575 nm (green to red), preferably 565 nm, or else about 400 nm to 453 nm (blue),
characterized in that
the receiver (18) is a photoresistor or a photodiode (18) or a phototransistor adapted to the wavelength, whereby there is a diaphragm positioned in front of the receiver so that essentially only parallel beams of light strike the receiver, and the signal corresponding to the inherent color of the plasma is automatically subtracted from the output signal of the receiver (18), and the differential signal is sent to an interrupter unit (16) through which the connection between the containers passes and which regulates the flow rate as a function of the differential signal, in particular interrupting the flow when a predefined limit value is reached.

9. Device for the fluid separation of whole blood as a mixture of fluids into individual, differently-colored blood constituents, the whole blood being packaged in flexible containers (5, 5'), especially bags, which are connected to each other by a connection (6) that is at least partially translucent, especially a flexible tube and, when force is applied, the blood constituents flow from one container through the translucent connection into another container, especially for the separation of a thrombocyte concentrate from buffy coat, with an optical-electric photometer unit (10, 18) that comprises a transmitter (10) and a receiver (18) for electromagnetic waves and past which the fluid flows inside the translucent connection, whereby the transmitter (10) of the photometer unit (10,18) is a light source such as a transmitting diode (10) or a light-emitting diode (LED) with a more or less monochromatic light having a wavelength within the range either from about 535 nm to 575 nm (green to red), preferably 565 nm, or else about 400 nm to 453 nm (blue),
characterized in that
the device has two optical-electric photometer units (10, 18), whereby there is a diaphragm in front of each of their receivers so that essentially only parallel beams of light strike the receiver, and the receiver (18) is a photoresistor or a photodiode (18) or a phototransistor adapted to the wavelength and, from the difference ΔV of the output signals of the receivers of the photometer units, a signal is derived that is sent to an interrupter unit (16) through which the connection between the containers passes and which regulates the flow rate as a function of the differential signal, in particular interrupting the flow when a predefined limit value is reached.

10. Device according to Claim 9, characterized in that
the photometer units (10, 18) are provided with a measuring bridge for equalization.

11. Device according to Claim 8 or 9, characterized by
a clamping block (7) into which the photometer unit is integrated and inside which the translucent connection (6) between the containers (5, 5') of the fluid passes, and the transmitter (10) and the receiver (18) are located in the vicinity of the connection, by a flow restrictor (12) likewise for restricting or regulating the flow of the fluid which is located in the vicinity downstream from the photometer unit and which generates an adjustable flow resistance for the fluid inside the connection (6).

12. Device according to Claim 8 or 9, characterized in that
the interrupter unit for clamping off the connection tube between two bags has a movably supported electromagnet (16) that can be actuated by the receiver (18) of the photometer unit.

13. Device according to Claim 8 or 9, characterized in that,
in order to distinguish between the various wavelengths, the receiver (18) has a corresponding bandwidth in the green-red range or blue range, for example, ±15% of the wavelength range.

14. Device according to Claim 8 or 9, characterized in that
the transmitter of the photometer unit is a white light lamp with a filter for generating a green and/or red and/or blue monochromatic light.

15. Device according to Claim 8 or 9, characterized in that
the clamping block (7) has a longitudinal groove (9) in which the tube (6) is laid and into which a crosswise groove (11) opens up and in which the flow restrictor (12) is located.

16. Device according to Claim 8 or 9, characterized in that
the flow restrictor is a wedge (12) that can be slid in the crosswise groove (11) for pinching the tube (6) to varying degrees.

17. Device according to Claim 8 or 9, characterized in that
the wedge (12) can be moved by means of a screw (21), for example, a micrometer screw (21), or mechanically by means of an electric motor, especially a stepping motor, and a shifting mechanism coupled to the wedge and to the electric motor.

18. Device according to Claim 8 or 9, characterized in that
the second bag (5') into which part of the mixture from the first bag (5) is being squeezed is suspended on a weighing system (19) in order to continuously ascertain the filled weight of the bag.

19. Device according to Claim 18, characterized in that
the weighing system has an electric value pick-up that sends an electric control signal corresponding to the changing weight of the bag (5') to the electric motor that is attached to the clamping block (7) and that acts upon the shifting mechanism in order to slide the wedge (12) back and forth in a controlled manner in the crosswise groove (11) of the clamping block (7).

20. Device according to Claim 8 or 9, characterized in that,
associated with the interrupter unit (8) as a clamping means for the tube (6), there is a mechanical opening cam (23) that opens the flow cross section of the tube (6) very slowly when it is activated.

21. Device according to Claim 20, characterized in that
the clamping unit is integrated into the interrupter unit (8) and the movable part of the electromagnet (16) is capable of opening very slowly.

22. Device according to Claim 21, characterized in that
the clamping unit is integrated into the flow restrictor (7) and the wedge (12) can additionally be moved as an opening cam by means of the electric motor (22).

## Revendications

1. Procédé pour séparer par fluage du sang complet sous forme de mélange de liquides, en constituants sanguins individuels de différentes couleurs, lequel est emballé dans des contenants souples (5,5'), notamment des sachets, qui sont reliés entre eux à l'aide d'une conduite de liaison (6) au moins partiellement transparente à la lumière, en particulier par un flexible, les constituants sanguins s'écoulant sous l'action d'une force de l'un des contenants vers l'autre via la conduite de liaison transparente à la lumière, notamment pour séparer un concentré de thrombocytes d'une couche leuco-plaquettaire, avec l'utilisation d'une unité photométrique opto-électrique (10,18) disposée à proximité d'une conduite de liaison transparente à la lumière et qui héberge un émetteur (10) et un récepteur (18) d'ondes électromagnétiques devant lesquels coule le liquide à l'intérieur de la conduite de liaison transparente à la lumière, en utilisant en tant qu'émetteur (10) de l'unité photométrique (10,18) une source lumineuse, telle qu'une diode émettrice (10) ou une diode lumineuse (LED) engendrant un rayonnement plus ou moins monochromatique d'une longueur d'onde comprise ou entre 535 nm et 575 nm environ (vert jusqu'à rouge), de préférence de 565 nm, ou entre 400 nm et 453 nm environ (bleu)
caractérisé en ce que
la lumière est diaphragmée devant le récepteur de sorte que, essentiellement, seulement des rayons lumineux parallèles tombent sur le récepteur et que le récepteur (18) est une photorésistance adaptée à la longueur d'onde ou une photodiode (18) ou un phototransistor, le signal correspondant à la couleur propre du plasma étant prélevé automatiquement du signal de sortie du récepteur (18) et le signal différentiel servant à commander une unité d'interruption (16), à travers laquelle passe la conduite de liaison entre les contenants et qui règle le débit, notamment qui arrête le flux lorsqu'une valeur limite prédéterminée est atteinte.

2. Procédé selon la revendication 1, caractérisé en ce que
une valeur maximum est prédéterminée pour la couleur propre du plasma et que la transformation de la préparation sanguine est interdite lorsque cette valeur est dépassée, de sorte que les préparations lipémiques et hémolytiques sont automatiquement exclues de la transformation.

3. Procédé selon l'une quelconque des revendications 1 ou 2, caractérisé en ce que,
pour augmenter la pureté du constituant sanguin séparé par compression, un autre signal de faible amplitude est prélevé du signal de sortie du récepteur en plus du signal correspondant à la couleur propre du plasma, le signal différentiel servant à régler le débit de l'unité d'interruption (16).

4. Procédé pour séparer par fluage du sang complet sous forme de mélange de liquides, en constituants sanguins individuels de différentes couleurs, lequel est emballé dans des contenants souples (5,5'), notamment des sachets, qui sont reliés entre eux à l'aide d'une conduite de liaison (6) au moins partiellement transparente à la lumière, en particulier par un flexible, les constituants sanguins s'écoulant sous l'action d'une force de l'un des contenants vers l'autre via la conduite de liaison transparente à la lumière, notamment pour séparer un concentré de thrombocytes d'une couche leuco-plaquettaire, avec l'utilisation d'une unité photométrique opto-électrique (10,18) disposée à proximité d'une conduite de liaison transparente à la lumière et qui héberge un émetteur (10) et un récepteur (18) d'ondes électromagnétiques devant lesquels coule le liquide à l'intérieur de la conduite de liaison transparente à la lumière, en utilisant en tant qu'émetteur (10) de l'unité photométrique (10,18) une source lumineuse, telle qu'une diode émettrice (10) ou une diode lumineuse (LED) engendrant un rayonnement plus ou moins monochromatique d'une longueur d'onde comprise ou entre 535 nm et 575 nm environ (vert jusqu'à rouge), de préférence de 565 nm, ou entre 400 nm et 453 nm environ (bleu), caractérisé en ce que
l'on utilise deux unités photométriques opto-électriques (10,18) distantes l'une de l'autre, la lumière étant diaphragmée devant le récepteur, de sorte que, essentiellement, seulement des rayons lumineux parallèles tombent sur le récepteur et que le récepteur (18) est une photorésistance adaptée à la longueur d'onde ou une photodiode (18) ou un phototransistor et q'un signal est prélevé de la différence ΔV des signaux de sortie des récepteurs des unités photométriques servant à régler le débit d'une unité d'interruption (16), à travers laquelle passe la conduite de liaison entre les contenants.

5. Procédé selon l'une quelconque des revendications 1 ou 4, caractérisé en ce que,
pour éviter que le liquide ne s'écoule par à-coups dans l'unité photométrique (10,18), la quantité de liquide débitée est réduite dans la phase initiale et la section de passage est dégagée très lentement de façon retardée dans le temps, en particulier de manière mécano-excentrique, jusqu'à ce que l'air se soit échappé.

6. Procédé selon l'une quelconque des revendications 1 ou 4, caractérisé en ce que
l'émetteur (10) de l'unité photométrique est commandé de façon cadencée.

7. Procédé selon l'une quelconque des revendications 1 ou 4, caractérisé en ce que,
pour la détection de thrombocytes et/ou de leucocytes, la lumière traversant la conduite de liaison, de préférence de courte longueur d'onde, est diaphragmée devant le récepteur (18), de sorte que, essentiellement, seulement des rayons lumineux parallèles tombent sur le récepteur (18), un rendement lumineux faible signifiant une concentration élevée de thrombocytes et/ou de leucocytes dans le liquide et vice-versa, en raison de la diffusion de la lumière sur les thrombocytes et/ou leucocytes.

8. Dispositif pour séparer par fluage du sang complet sous forme de mélange de liquides, en constituants sanguins individuels de différentes couleurs, lequel est emballé dans des contenants souples (5,5'), notamment des sachets, qui sont reliés entre eux à l'aide d'une conduite de liaison (6) au moins partiellement transparente à la lumière, en particulier par un flexible, les constituants sanguins s'écoulant sous l'action d'une force de l'un des contenants vers l'autre va la conduite de liaison transparente à la lumière, notamment pour séparer un concentré de thrombocytes d'une couche leuco-plaquettaire, comprenant une unité photométrique opto-électrique (10,18) qui héberge un émetteur (10) et un récepteur (18) d'ondes électromagnétiques devant lesquels coule le liquide à l'intérieur de la conduite de liaison transparente à la lumière, l'émetteur de l'unité photométrique étant une source lumineuse (10), telle qu'une diode émettrice (10) ou une diode lumineuse (LED) engendrant un rayonnement plus ou moins monochromatique d'une longueur d'onde comprise ou entre 535 nm et 575 nm environ (vert jusqu'à rouge), de préférence de 565 nm, ou entre 400 nm et 453 nm environ (bleu), caractérisé en ce que
le récepteur (18) est une photorésistance adaptée à la longueur d'onde ou une photodiode (18) ou un phototransistor et qu'un diaphragme est disposé devant le récepteur de sorte que, essentiellement, seulement des rayons lumineux parallèles tombent sur le récepteur, le signal correspondant à la couleur propre du plasma étant prélevé automatiquement du signal de sortie du récepteur (18) et le signal différentiel étant transmis à une unité d'interruption (16) à travers laquelle passe la conduite de liaison entre les contenants et qui règle le débit en fonction du signal différentiel, notamment qui arrête le flux lorsqu'une valeur limite prédéterminée est atteinte.

9. Dispositif pour séparer par fluage du sang complet sous forme de mélange de liquides, en constituants sanguins individuels de différentes couleurs, lequel est emballé dans des contenants souples (5,5'), notamment des sachets, qui sont reliés entre eux à l'aide d'une conduite de liaison (6) au moins partiellement transparente à la lumière, en particulier par un flexible, les constituants sanguins s'écoulant sous l'action d'une force de l'un des contenants vers l'autre via la conduite de liaison transparente à la lumière, notamment pour séparer un concentré de thrombocytes d'une couche leuco-plaquettaire, comprenant une unité photométrique opto-électrique (10,18) qui héberge un émetteur (10) et un récepteur (18) d'ondes électromagnétiques devant lesquels coule le liquide à l'intérieur de la conduite de liaison transparente à la lumière, l'émetteur de l'unité photométrique étant une source lumineuse (10), telle qu'une diode émettrice (10) ou une diode lumineuse (LED) engendrant un rayonnement plus ou moins monochromatique d'une longueur d'onde comprise ou entre 535 nm et 575 nm environ (vert jusqu'à rouge), de préférence de 565 nm, ou entre 400 nm et 453 nm environ (bleu), caractérisé en ce que
le dispositif présente deux unités photométriques opto-électriques (10,18), que devant chacun de leurs récepteurs est disposé un diaphragme de sorte que, essentiellement, seulement des rayons lumineux parallèles tombent sur le récepteur, que le récepteur (18) est une photorésistance adaptée à la longueur d'onde ou une photodiode (18) ou un phototransistor et qu'un signal est prélevé de la différence ΔV des signaux de sortie des récepteurs et transmis à une unité d'interruption (16) à travers laquelle passe la conduite de liaison entre les contenants et qui règle le débit en fonction du signal différentiel, notamment qui arrête le flux lorsqu'une valeur limite prédéterminée est atteinte.

10. Dispositif selon la revendication 9, caractérisé en ce que
les unités photométriques (10,18) sont dotées d'un pont de mesure pour l'alignement.

11. Dispositif selon l'une quelconque des revendications 8 ou 9, caractérisé en ce que
ledit dispositif comprend un bloc de serrage (7), dans le quel est intégrée l'unité photométrique et à l'intérieur duquel passe la conduite de liaison (6) transparente à la lumière entre les contenants (5,5') du liquide et l'émetteur (10) et le récepteur (18) se trouvant à proximité de la conduite de liaison, ainsi qu'une unité de limitation du débit (12) pour imiter ou régler le débit du liquide, qui est disposée à proximité et en aval de l'unité photométrique et qui constitue une résistance à l'écoulement du liquide dont le débit est réglable à l'intérieur de la conduite de liaison (6).

12. Dispositif selon l'une quelconque des revendications 8 ou 9, caractérisé en ce que
l'unité d'interruption servant à comprimer le flexible qui relie deux sachets présente un électro-aimant (16) logé de façon mobile, qui peut être commandé par le récepteur (18) de l'unité photométrique.

13. Dispositif selon l'une quelconque des revendications 8 ou 9, caractérisé en ce que,
pour distinguer les différentes longueurs d'ondes, le récepteur (18) possède une largeur de bande correspondante respectivement dans la gamme vertrouge et dans la gamme bleue, par exemple de ± 15% de la gamme d'ondes.

14. Dispositif selon l'une quelconque des revendication 8 ou 9, caractérisé en ce que
l'émetteur de l'unité photométrique est une lampe à lumière blanche avec un filtre pour générer un rayonnement monochromatique vert et/ou rouge et/ou bleu.

15. Dispositif selon l'une quelconque des revendications 8 ou 9, caractérisé en ce que
le bloc de serrage (7) présente un canal longitudinal (9), dans lequel passe le flexible (6) et dans lequel débouche un canal transversal (11), dans lequel est disposée l'unité de limitation du débit (12).

16. Dispositif selon l'une quelconque des revendications 8 ou 9, caractérisé en ce que
l'unité de limitation du débit est une clavette (12) disposée dans le canal transversal (11) et pouvant se déplacer pour comprimer plus ou moins fortement le flexible (6).

17. Dispositif selon l'une quelconque des revendications 8 ou 9, caractérisé en ce que
la clavette (12) peut être déplacée par intervention manuelle au moyen d'une vis (21) telle qu'une vis micrométrique (21), ou à l'aide d'un moteur électrique, notamment d'un moteur pas-à-pas, et par un dispositif de déplacement coupé à la clavette et au moteur électrique.

18. Dispositif selon l'une quelconque des revendications 8 ou 9, caractérisé en ce que
le deuxième sachet (5'), dans lequel est introduite par pression une partie du mélange du premier sachet (5), est suspendu à n dispositif de pesée (19) permettant de déterminer en continu le poids de remplissage du sachet.

19. Dispositif selon la revendication 18, caractérisé en ce que
le dispositif de pesée présente un transmetteur électrique de la valeur de réglage qui, en fonction du poids du sachet (5') transmet un signal électrique de réglage au moteur électrique qui est fixé sur le bloc de serrage (7) et agit sur le dispositif de déplacement en faisant avancer ou reculer de façon réglée la clavette (12) dans le canal transversal (11) du bloc de serrage (7).

20. Dispositif selon l'une quelconque des revendications 8 ou 9, caractérisé en ce que
un excentrique d'ouverture mécanique (23), assigné à l'unité d'interruption (8), est disposé en tant qu'unité de compression du flexible (6) et qui, lorsqu'il est actionné, ouvre très lentement la section du passage du flexible (6).

21. Dispositif selon la revendication 20, caractérisé en ce que
l'unité de compression se trouve intégrée à l'unité d'interruption (8) et que la partie mobile de l'électro-aimant (16) est en mesure d'ouvrir très lentement.

22. Dispositif selon la revendication 21, caractérisé en ce que
l'unité de compression se trouve intégrée à l'unité de limitation du débit (7) et que, de plus, la clavette (12) sert d'excentrique d'ouverture et peut être déplacée à l'aide du moteur électrique (22).
